# EUROPEAN PATENT SPECIFICATION

(11) **EP 0 675 830 B1**
(45) Date of publication and mention of the grant of the patent: **15.03.2000**
(21) Application number: 94904897.9
(22) Date of filing: 29.12.1993
(51) Int. Cl.: B65D 51/00

(54) **THIN DIAPHRAGM STOPPER FOR BLUNT ENTRY DEVICE**
DÜNNER MEMBRANSTOPFEN FÜR EINE STUMPFE DURCHDRINGUNGSVORRICHTUNG.
BOUCHON A FINE MEMBRANE POUR DISPOSITIF PENETRANT NON POINTU

(30) Priority: 30.12.1992 US 998232; 23.12.1993 US 168481
(43) Date of publication of application: 11.10.1995
(73) Proprietor: ABBOTT LABORATORIES, Abbott Park, Illinois 60064-3500 (US)
(72) Inventor: NORVILLE, James, D., Rocky Mount, NC 27801 (US); FERNANDO, Juan, V., Tampa, FL 33624 (US); ELIAS, Allen, M., Mundelein, IL 60060 (US)
(74) Representative: Modiano, Guido, Dr.-Ing.
(86) International application number: US9312614
(87) International publication number: WO9415850

(56) References cited:
- EP-A- 0 086 251
- EP-A- 0 120 353
- EP-A- 0 509 281
- WO-A-94/03373
- GB-A- 2 106 084
- US-A- 4 582 207
- US-A- 4 741 446
- US-A- 4 915 243
- US-A- 4 967 919
- US-A- 5 100 010

## Description

### FIELD OF THE INVENTION

The present invention relates generally to a closure for sealing an open-necked container or the like and more particularly to a resilient closure having a thin diaphragm portion for an associated blunt entry device to be inserted therethrough.

### BACKGROUND OF THE INVENTION

Closures such as resilient stoppers and reseal members are widely used in the health care environment to seal solution containers such as vials or the like which require the insertion of a needle or cannula to add or withdraw solution from the container. Closures such as stoppers are used on containers or vials for storage of sterile parenteral solutions and the like. Closures such as reseals are used at fluid port sites for medical tubing fluid communication sets such as at Y-sites for intravenous (IV) administration sets.

The closures or stoppers currently in use for small volume parenteral fluid containers such as vials are usually pierced by the sharp needle of a syringe or a vial adapter. The reseals in IV administration sets are usually pierced by a syringe needle or a needle connector. Thus the inserted sharp entry device allows the stored solution to be added or withdrawn in a sterile manner.

Closures such as stoppers are typically fabricated of a resilient material such as natural rubber or a manufactured elastomer. Reseals are traditionally fabricated of natural rubber or a manufactured elastomer. Both of these materials, as well as others, have an inherent ability to retain their original geometric shape after the closure material has been punctured.

Within the last few years, the health care industry has become acutely aware of the consequences of "accidental" needle stick. The industry has responded by promoting safety in the performance of invasive medical procedures. To promote safe performance of daily health care procedures, sharp entry devices (i.e."sharps"), are being used less. The use of blunt, relatively unsharpened needles and cannulas is becoming more widespread as a result of the growing anti-sharp needle philosophy.

In connection with the use of blunt entry devices is the concern about the required piercing force needed to insert the blunt device through a closure such as a stopper or a reseal. The materials suitable for use as a closure, that is having high resilient properties, tend to have a relatively high and undesirable tensile strength which resists puncture by blunt devices.

There are known closure constructions with diaphragm portions that might accommodate a blunt entry device. However, these known closures are typically manufactured for a special use and have inherent limitations for general use, such as expense, use with only special entry devices, risk of contamination or lack of resealability, among others.

For example, U.S. Patent No. 2,906,423 to Sandhage discloses a stopper formed of a low tear-resistant rubber, with a conical well in the top face extending partway into the plug portion of the stopper. A cut slit extends partway from the apex of the conical well towards the bottom face of the stopper. This construction provides a moisture proof closure as well as a closure that can be entered by forcing a blunt plastic needle therethrough. However, the stopper of Sandhage is difficult to swab with an antiseptic agent prior to entry by a blunt entry device because of the concavity and slit on the top entry surface. Therefore, in many situations this type of reseat may increase the risk of introducing potential contaminants into the vial. Also the expense of manufacturing and controlling tolerances and quality for the multiple steps in the molding, slitting and cleaning of the Sandhage stopper would be prohibitive for any product other than as a special closure for which manufacturing cost is relatively unimportant.

U.S. Patent No. 5,060,812 to Ogle discloses the use of a transverse slot or slots to thin a stopper diaphragm for entry by a male luer. However the manufacture process of the Ogle stopper is complex and thus expensive. Also the concavity of the top surface of the Olge stopper creates potential contamination problems even with swabbing.

U.S. Patent No. 3,653,528 to Wimmer discloses a stopper with a thin diaphragm portion including an indentation in the top surface. The configuration of the Wimmer stopper increases the swabbing problems and introduces further difficulties for reseating. Entry by a blunt device would tear the diaphragm along the conical wall portion. There would be little radial compressive forces to assist in sealing around the inserted blunt entry device or in reseating the diaphragm once the entry device is withdrawn.

Further a closure according to the preamble of claim 1 is disclosed in document EP-A-120 353.

Accordingly, a reseal member is disclosed in which the diaphragm portion has a preferential configuration to facilitate the insertion of a blunt needle or cannula therethrough. Also the upper and lower diaphragm surfaces are continuous and unbroken so as to inhibit contamination.

### SUMMARY OF THE INVENTION

The present invention related to an improved closure for sealing an open-ended container and usable with a blunt entry device. The closure includes a resilient body having a central diaphragm portion, a peripheral flange portion, and a hollow plug portion. The flange portion radially surrounds the diaphragm portion and has a continuous juncture with the diaphragm portion. The central diaphragm portion has a thickness that accommodates the insertion of the blunt entry device therethrough. The flange portion is arranged to abut the open neck of the container. The plug portion is constructed to depend from the continuous juncture of the diaphragm and the flange portions and sealingly engage the neck of the container. The diaphragm portion has a continuous and unbroken upper surface and a continuous and unbroken curvilinear lower surface. The lower surface has a radius of curvature in the range of 0.4 to 0.6 times the diameter of the central diaphragm portion.

The peripheral flange portion of the present invention preferably is a flat annular ring having a predetermined thickness and surrounding the diaphragm portion. Preferably, the flange portion has an upper surface in general alignment with the upper surface of the diaphragm portion, Preferably, at least a portion of the thickness of the diaphragm portion is less than the predetermined thickness of the flange portion. The lower surface of the diaphragm portion may have a concave shape with a central minimal thickness.

During use with a blunt entry device, the diaphragm portion is inwardly deformable in tensile stress by the blunt entry device prior to failure. Advantageously, the diaphragm portion tends to center the blunt entry device toward the central minimal thickness.

Other features and advantages of the present invention will be apparent from the detailed description, the accompanying drawings and the appended claims.

### BRIEF DESCRIPTIONS OF THE DRAWINGS

Figure 1 is a perspective view of a standard solution vial with a ferrule securing a closure member such as a resilient stopper;
Figure 1A, B is a cross-sectional view of a standard resilient stopper according to the prior art;
Figure 2 is a cross-sectional view of a resilient stopper according to the present invention;
Figures 3 is a cross-sectional view of the resilient stopper according to the present invention after complete assembly;
Figures 4 is a cross-sectional view of the resilient stopper according to the present invention during a second stage of assembly;
Figures 5 is a cross-sectional view of the resilient stopper according to the present invention during an initial stage of use;
Figures 6 is a cross-sectional view of the resilient stopper according to the present invention during use;
Figures 7 is a cross-sectional view of the resilient stopper according to the present after the inserted blunt device has been withdrawn;
Figures 8 is a cross-sectional view of the stopper according to the present invention showing a blunt entry device being applied off center ; and
Figures 9 is a cross-sectional view of the stopper according to the present invention showing a blunt entry device being centered by the present invention.

### DETAILED DESCRIPTION OF THE PREFERRED EMBODIMENTS

While the present invention is susceptible of embodiment in various forms, there is shown in the drawings and will hereinafter be described a presently preferred embodiment with the understanding that the present disclosure is to be considered an example of the present invention and is not intended to limit the invention to the specific embodiments illustrated.

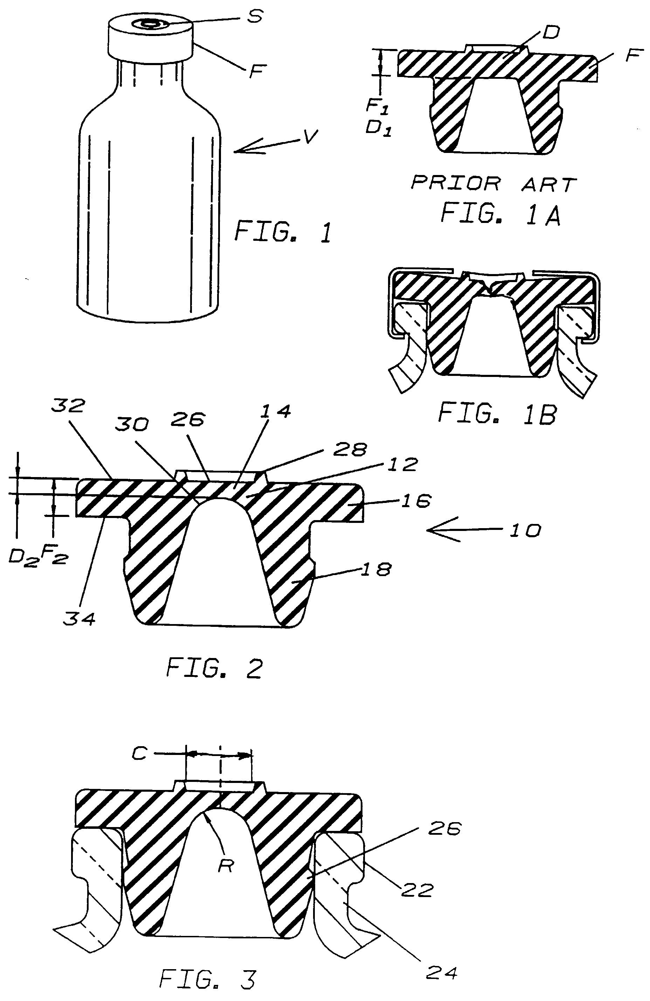

With reference now to the drawings, there is illustrated in Figure 1 a perspective view of a standard glass vial V for storage of a medical solution. A closure member S and metal ferrule F are sealingly positioned in and over the neck of the vial to seal the open end of the vial.

Figure 1A shows a cross section of a traditional closure member such as a standard thick diaphragm stopper that is well known in the prior art. The stopper diaphragm portion D₁ which is piercable by a sharp entry device has a thickness that is approximately equal in thickness to the thickness of the stopper flange portion F₁. Conventionally, a sharp entry device must be used to communicate with the solution in the interior of a vial that is closed by a traditional thick diaphragm stopper. A blunt entry device requires excessive force to pierce a traditional thick diaphragm stopper.

Referring now to Figures 2 and 3, a closure 10 according to the present invention is shown. The closure 10 is preferably molded of a resilient material that is medically acceptable and compatible ( i.e. nonreactive or inert) with the medical solution to be contained in the vial. The stopper includes a central diaphragm portion 14, a peripheral flange portion 16, and a hollow plug portion 18. The flange portion 16 radially surrounds the central diaphragm portion 14 and has a continuous juncture with the diaphragm portion. The central diaphragm portion 14 has a thickness D₂ that accommodates the insertion of a blunt entry device therethrough. The flange portion 16 is arranged to abut the open neck 22 of the container. The plug portion 18 is constructed to depend from the continuous juncture of the central diaphragm portion 14 and the radially peripheral flange portion 16. The plug portion is frustoconical in shape and the skirt tapers outward to sealingly engage the neck 22 of the container 24. The diaphragm portion 14 has a continuous and unbroken upper surface or target area 26 delimited by a raised annular lip 28. The diaphragm portion also has a continuous and unbroken lower surface 30 closing off the end of the hollow plug portion 18.

The peripheral flange portion 16 of the present invention preferably is a flat annular ring having a predetermined thickness F₂ and continuously surrounding the central diaphragm portion 14. The flange portion 16 has an upper surface 32 in general alignment with the upper surface 26 of the diaphragm portion 14 and a parallel lower surface 34. At least a center portion of the variable thickness of the diaphragm portion 14 is less than the predetermined thickness F₂ of the peripheral flange portion 16. Furthermore, the thickness D₂ of the central diaphragm portion 14 is variable because it is partially determined by a curvilinear tapering lower surface 30 of the central diaphragm portion 14. The lower surface 30 of the diaphragm portion preferably has a concave shape with a central minimal thickness D₂.

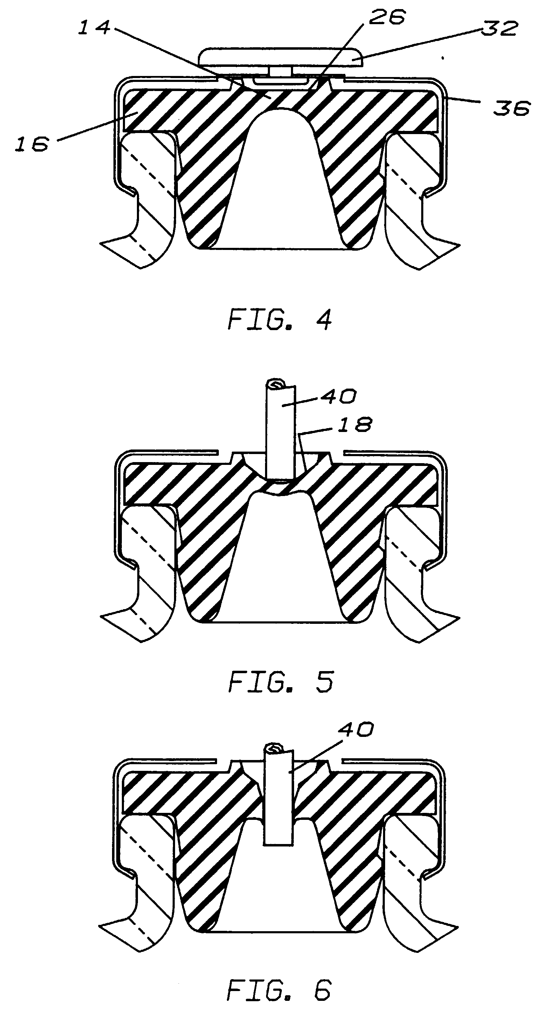

In Figure 4, a metal ferrule 36 with a removable plastic cap element 38 is assembled by crimping the ferrule to the stopper 10 and the vial 24, creating axial compressive forces in the peripheral flange portion 16 of the stopper. If the central diaphragm portion 14 is not adequately supported, the target area 26 of the central diaphragm portion may concave or indent (i.e. wrinkle) when the metal ferrule compresses the flange portion 16. For example the axial force exerted by the assembly of the ferrule 36 with the resilient stopper 10 tends to cause buckling in the target area 26 if the diaphragm portion is not properly supported. Wrinkling is unacceptable because the wrinkle or indentation can create the false impression that the vial has been tampered with or has already been used. Even minor wrinkling is unacceptable as the wrinkles make the upper stopper surface or target area 26 difficult to swab with an antiseptic agent.

As best seen in Figure 5, the central diaphragm portion 14 is initially inwardly deformable in tensile stress by a blunt entry device. After a small resistance, the diaphragm is pierced and seals around the inserted blunt cannula or blunt needle 40 as seen in Figure 6. The force for the blunt entry device to penetrate the thin diaphragm stopper compares favorably with the force required by a sharp needle to pierce a traditional thick diaphragm stopper.

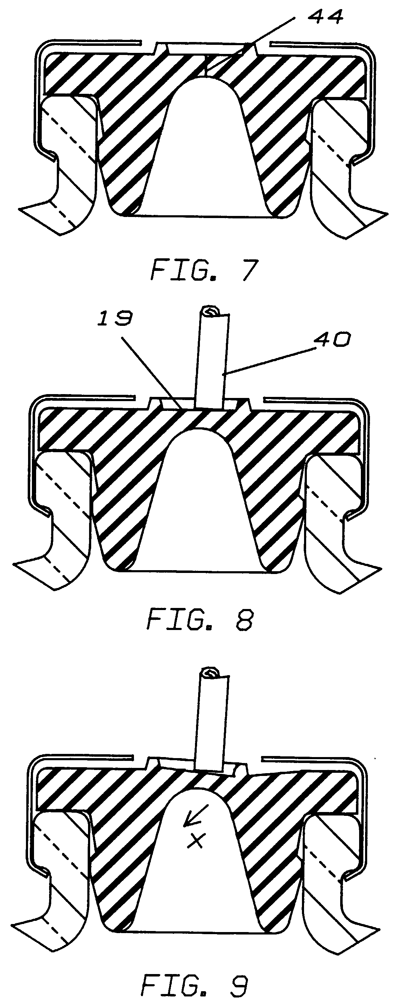

As shown in Figure 7, the pierced diaphragm portion tends to seal back together when the blunt entry device is removed. Advantageously, as shown in Figures 8 and 9, the central diaphragm portion or target area 26 of the present invention tends to center the blunt entry device 40 toward the central minimal thickness.

In the preferred embodiment of the present invention, the thickness D₂ of the diaphragm portion 14 is substantially less than the thickness F₂ of the flange portion 16. D₂ is preferably approximately 0.03 inches (0.762 mm), while F2 is about 0.050 inches (1.27 mm).

In one embodiment of the invention according to Figures 8 and 9, the central minimal thickness of the diaphragm portion is 0.030 inches (0.762 mm), the diameter of the upper surface of the diaphragm portion and the plug concavity is 0.100 inches (2.54 mm), and the radius at the tip of the plug concavity is 0.053 inches (1.35 mm).

The lower surface 30 of the diaphragm portion 14 is curvilinearly tapered. This construction provides support to prevent wrinkling. This construction also provides radial compressive forces to aid resealing of the pierced stopper. Finally the rounded diaphragm surfaces 30 facilitates cleaning and sterilization of the stopper interior surfaces that will contact the enclosed solution prior to assembly.

The ratio of the radius R in the inner rounded surface of the diaphragm to the diameter of the target area C and plug concavity is in the range of 0.4 to 0.6.

The stopper configuration of Figure 2 can be readily molded in large quantities for economical manufacture. The present invention provides the advantage of a thin diaphragm stopper that is accessible with a blunt entry device while also providing a smooth outer surface for swabbing. Also the construction avoids a wrinkled or tampered appearance that might occur during assembly by providing a structural resistance to the application of the forces during assembly.

The stopper body of the present invention is moldable of an elastomeric material in large batch quantities in a single step process. Further manufacturing steps are not required. Cleaning and sterilization of the molded stopper prior to assembly can be readily accomplished since the surfaces are exposed and mostly curvilinear. The surfaces do not have discontinuous surface breaks, especially in the hollow interior portion of the plug which is in fluid contact. Thus manufacturing and assembly of the stopper according to the present invention is straightforward not complex.

It is understood that the above descriptions are made only by way of example and are not intended to limit the scope of the invention. Many variations and modifications can be carried out without departing from the invention as defined in the claims. For example, the thin diaphragm portion is alternatively curvilinearly tapered on both upper and lower surfaces. Accordingly, the claims are not intended to be limited by the above specific embodiment.

## Claims

1. A closure for sealing an open neck of a container and for use with a blunt entry device, the closure comprising:
a resilient body having a central diaphragm portion, a peripheral flange portion having a continuous juncture with the diaphragm portion and surrounding the diaphragm portion, and a hollow plug portion, the central diaphragm portion having a thickness for insertion of the blunt entry device therethrough, the flange portion arranged to abut the open neck of the container, and the plug portion constructed to depend from the continuous juncture of the diaphragm and the flange portions and sealingly engage the neck of the container, the diaphragm portion having a continuous and unbroken upper surface and a continuous and unbroken curvilinear lower surface, caracterized in that said lower surface has a radius of curvature in the range of 0.4 to 0.6 times the diameter of the central diaphragm portion.

2. The closure of claim 1 wherein the peripheral flange portion is a flat annular ring having a predetermined thickness and surrounding the diaphragm portion, the flange portion having an upper surface in general alignment with the upper surface of the diaphragm portion.

3. The closure of claim 2 wherein at least a portion of the thickness of the diaphragm portion is less than the predetermined thickness of the flange portion.

4. The closure of claim 1 wherein the lower surface of the diaphragm portion has a concave shape with a central minimal thickness.

5. The closure of claim 4 wherein the diaphragm portion is inwardly deformable in tensile stress by the blunt entry device prior to failure.

6. The closure of claim 5 wherein the inward deformation of the diaphragm portion tends to center the blunt entry device toward the central minimal thickness.

7. The closure of claim 6 wherein the central minimal thickness of the diaphragm portion is 0.030 inches (0.762 mm), the diameter of the upper surface of the diaphragm portion and the plug concavity is 0.100 inches (2.54 mm), and the radius at the tip of the plug concavity is 0.053 inches (1.35 mm).

## Patentansprüche

1. Ein Verschluß zum Abdichten eines offenen Halses eines Behälters und zur Verwendung mit einer stumpfen Durchdringungsvorrichtung, wobei der Verschluß folgendes umfaßt:
einen elastischen Körper, der einen zentralen Membranabschnitt, einen peripheren Flanschabschnitt, der über eine kontinuierliche Verbindungsstelle mit dem Membranabschnitt verfügt und den Membranabschnitt umgibt, und einen hohlen Pfropfabschnitt aufweist, wobei der zentrale Membranabschnitt eine Dicke zum Einfügen der stumpfen Durchdringungsvorrichtung dadurch aufweist, wobei der Flanschabschnitt angeordnet ist, um gegen den offenen Hals des Behälters zu stoßen, und wobei der Pfropfabschnitt aufgebaut ist, um von der kontinuierlichen Verbindungsstelle der Membran- und Flanschabschnitte abzuhängen und mit dem Hals des Behälters abdichtend im Eingriff zu stehen, wobei der Membranabschnitt über eine kontinuierliche und nicht unterbrochene obere Oberfläche und eine kontinuierliche und nicht unterbrochene, krummlinige untere Oberfläche verfügt, dadurch gekennzeichnet, daß die untere Oberfläche einen Krümmungsradius hat, der im Bereich von 0,4 bis 0,6 mal dem Durchmesser des zentralen Membranabschnitts liegt.

2. Der Verschluß nach Anspruch 1, worin der periphere Flanschabschnitt ein flacher, ringförmiger Ring ist, der eine vorbestimmte Dicke aufweist und den Membranabschnitt umgibt, wobei der Flanschabschnitt eine mit der oberen Oberfläche des Membranabschnitts allgemein ausgerichtete obere Oberfläche aufweist.

3. Der Verschluß nach Anspruch 2, worin mindestens ein Abschnitt der Dicke des Membranabschnitts dünner ist als die vorbestimmte Dicke des Flanschabschnitts.

4. Der Verschluß nach Anspruch 1, worin die untere Oberfläche des Membranabschnitts konkavförmig mit einer zentralen minimalen Dicke ist.

5. Der Verschluß nach Anspruch 4, worin der Membranabschnitt, vor seinem Versagen, durch die stumpfe Durchdringungsvorrichtung zugbeansprucht nach innen verformbar ist.

6. Der Verschluß nach Anspruch 5, worin die Innenverformung des Membranabschnitts dazu neigt, die stumpfe Durchdringungsvorrichtung in Richtung der zentralen minimalen Dicke zu zentrieren.

7. Der Verschluß nach Anspruch 6, worin die zentrale minimale Dicke des Membranabschnitts 0,030 Zoll (0,762 mm), der Durchmesser der oberen Oberfläche des Membranabschnitts und die Pfropfkonkavität 0,100 Zoll (2,54 mm) und der Radius an der Spitze der Pfropfkonkavität 0,053 Zoll (1,35 mm) beträgt.

## Revendications

1. Dispositif de fermeture pour fermer de façon étanche une embouchure ouverte ouvert d'un récipient et destiné à être utilisé avec un dispositif de pénétration non pointu, le dispositif de fermeture comprenant :
un corps élastique possédant une partie centrale formant membrane, une partie formant bride périphérique possédant une jonction continue avec la partie formant membrane et entourant la partie formant membrane, et une partie formant bouchon creux, la partie centrale formant membrane possédant une épaisseur permettant l'insertion et la traversée du dispositif de pénétration non pointu, la partie formant bride étant disposée de manière à être en butée autour de l'embouchure ouverte du récipient, et la partie formant bouchon étant agencée de manière à s'étendre à partir de la jonction continue de la membrane et de la partie formant bride et s'appliquant d'une manière étanche contre l'embouchure du récipient, la partie formant membrane possédant une surface supérieure continue ininterrompue et une surface inférieure curviligne continue et ininterrompue,
caractérisé en ce que ladite surface inférieure possède un rayon de courbure compris dans la gamme de 0,4 à 0,6 fois le diamètre de la partie centrale formant membrane.

2. Dispositif de fermeture selon la revendication 1, dans lequel la partie formant bride périphérique est une bague annulaire plate possédant une épaisseur prédéterminée et entourant la partie formant membrane, la partie formant bride ayant une surface supérieure qui est d'une manière générale alignée avec la partie supérieure de la partie formant membrane.

3. Dispositif de fermeture selon la revendication 2, dans lequel au moins une partie de l'épaisseur de la partie formant membrane est inférieure à l'épaisseur prédéterminée de la partie formant bride.

4. Dispositif de fermeture selon la revendication 1, dans lequel la surface inférieure de la partie formant membrane possède une forme concave ayant une épaisseur centrale minimale.

5. Dispositif de fermeture selon la revendication 4, dans lequel la partie formant membrane est déformable vers l'intérieur sous l'effet d'une contrainte de traction par le dispositif de pénétration non pointu avant sa défaillance.

6. Dispositif de fermeture selon la revendication 5, dans lequel la déformation intérieure de la partie formant membrane tend à centrer le dispositif de pénétration non pointu en direction de la zone centrale d'épaisseur minimale.

7. Dispositif de fermeture selon la revendication 6, dans lequel l'épaisseur minimale au centre de la partie formant membrane est égale à 0,030 pouce (0,762 mm), le diamètre de la surface supérieure de la partie formant membrane et de la partie concave du bouchon est égale à 0,100 pouce (2,54 mm) et le rayon au sommet de la concavité du bouchon est égal à 0,053 pouce (1,35 mm).
